# EUROPEAN PATENT APPLICATION

(11) **EP 1 634 891 A1**
(43) Date of publication of application: **15.03.2006**
(21) Application number: 04734616.8
(22) Date of filing: 24.05.2004
(51) Int. Cl.: C07K 14/195, C12N 9/88

(54) **CHONDROITIN AC LYASE CRYSTAL**

(30) Priority: 22.05.2003 US 472689 P
(71) Applicant: SEIKAGAKU CORPORATION, Chuo-ku, Tokyo 103-0023 (JP)
(72) Inventor: LUNIN, Vladimir V. Biotechn. Res. Inst. National, Montreal, Quebec HP4P2R2 (CA); CYGLER, Miroslaw Biotechn. Res. Institute National, Montreal, Quebec HP4P2R2 (CA)
(74) Representative: Woods, Geoffrey Corlett
(86) International application number: PCT/JP2004/007432
(87) International publication number: WO 2004/104034

(57) **Abstract**

Crystals of chondroitin AC lyase originated from an *Arthrobacter* bacterium, which belong to the monoclinic system space group P2₁ substantially with unit cell dimensions of a = 57.6 Å, b = 85.5 Å, c = 80.5 Å, β = 106.9° and contain one molecule in the asymmetric unit, and a protein having an activity of chondroitin AC lyase and an amino acid sequence Asn-Trp-Trp-(Xaa)₇-Arg-(Xaa)₃₄₋Gln-(Xaa)₄-Arg- (Xaa)₈-Asn- (Xaa)₄₉-His- (Xaa)₈-Tyr- (Xaa)₅₃₋Arg- (Xaa)₃-Arg- (Xaa)₂- (Asn or Asp) - (Xaa)₁₀₆-Asn- (Xaa)₅₄-Trp (Xaa represents an arbitrary amino acid residue, and the numerals represent numbers of residues).

SEQUENCE LISTING

<110> Seikagaku Corporation

<120> Chondroitin AC Lyase Crystals

<130> SE04003PCT

<160> 2

<170> PatentIn version 3.2

<210> 1
<211> 757
<212> PRT
<213> Arthrobacter aurescens

<400> 1

Glu Ala Glu Pro Gly Ala Ala Glu Phe Ala Ala Leu Arg Asn Arg Trp
1               5                   10                  15

Val Asp Gln Ile Thr Gly Arg Asn Val Ile Gln Ala Gly Asp Pro Asp
            20                  25                  30

Phe Ala Lys Ala Ile Thr Ala Leu Asn Asn Lys Ala Ala Asp Ser Leu
        35                  40                  45

Ala Lys Leu Asp Val Lys Lys Gly Arg Thr Ser Val Phe Thr Asp Leu
    50                  55                  60

Ser Leu Ala Lys Asp Ala Glu Met Val Thr Thr Tyr Thr Arg Leu Ser
65                  70                  75                  80

Gln Leu Ala Thr Ala Trp Ala Thr Pro Thr Ala Ala Val Phe Gly Asp
                85                  90                  95

Ala Ala Val Leu Ala Ala Ile Lys Ala Gly Leu Ala Asp Ala Asn Thr
            100                 105                 110

Leu Cys Tyr Asn Asp Arg Lys Glu Glu Val Gly Asn Trp Trp Ser Trp
        115                 120                 125

Glu Ile Gly Val Pro Arg Ala Leu Ala Asp Ala Met Val Leu Leu His
    130                 135                 140

Ala Glu Leu Ser Ala Ala Glu Arg Thr Ala Tyr Cys Ala Ala Ile Asp
145                 150                 155                 160

His Phe Val Pro Asp Pro Trp Leu Gln Phe Pro Pro Lys Arg Gly Lys
                165                 170                 175

Ile Thr Ser Val Gly Ala Asn Arg Val Asp Leu Cys Gln Gly Ile Ile
            180                 185                 190

Ile Arg Ser Leu Ala Gly Glu Asp Pro Thr Lys Leu Asn His Ala Val
        195                 200                 205

Ala Gly Leu Ser Gln Val Trp Gln Tyr Val Thr Ser Gly Asp Gly Ile
    210                 215                 220

Phe Arg Asp Gly Ser Phe Ile Gln His Ser Thr Thr Pro Tyr Thr Gly
225                 230                 235                 240

Ser Tyr Gly Val Val Leu Leu Thr Gly Leu Ser Lys Leu Phe Ser Leu
                245                 250                 255

Leu Gly Gly Thr Ala Phe Glu Val Ser Asp Pro Thr Arg Ser Ile Phe
            260                 265                 270

Phe Asp Ala Val Glu Gly Ser Phe Ala Pro Val Met Ile Asn Gly Ala
        275                 280                 285

Met Ala Asp Ala Val Arg Gly Arg Ser Ile Ser Arg Glu Ala Asn Thr
    290                 295                 300

Gly Tyr Asp Leu Gly Ala Ser Ala Ile Glu Ala Ile Leu Leu Leu Ala
305                 310                 315                 320

Arg Ala Met Asp Pro Ala Thr Ala Ala Arg Trp Arg Gly Leu Cys Ala
                325                 330                 335

Gly Trp Ile Ala Arg Asp Thr Tyr Arg Pro Ile Leu Asn Ser Ala Ser
            340                 345                 350

Val Pro Arg Thr Ala Leu Val Lys Gln Leu Glu Ala Thr Gly Val Ala
        355                 360                 365

Pro Val Ala Glu Ala Thr Gly His Lys Leu Phe Pro Ala Met Asp Arg
    370                 375                 380

Thr Met His Arg Gly Pro Gly Trp Ala Leu Ser Leu Ala Leu Ser Ser

385                 390                 395                 400

Asn Arg Ile Ala Trp Tyr Glu Cys Gly Asn Gly Glu Asn Asn Arg Gly
                405                 410                 415

Tyr His Thr Gly Ser Gly Met Thr Tyr Phe Tyr Thr Ser Asp Leu Gly
            420                 425                 430

Gln Tyr Asp Asp Ala Phe Trp Ala Thr Ala Asn Tyr Asn Arg Leu Pro
        435                 440                 445

Gly Ile Thr Val Asp Thr Thr Pro Leu Pro Asp Lys Val Glu Gly Gln
    450                 455                 460

Trp Gly Ala Ala Val Pro Ala Asp Glu Trp Ser Gly Ala Thr Ala Leu
465                 470                 475                 480

Gly Glu Val Ala Ala Val Gly Gln His Leu Val Gly Pro Gly Arg Thr
                485                 490                 495

Gly Leu Thr Ala Arg Lys Ser Trp Phe Val Ser Gly Asp Val Thr Val
            500                 505                 510

Cys Leu Gly Ala Asp Ile Ser Thr Ala Ser Gly Ala Lys Val Glu Thr
        515                 520                 525

Ile Val Asp His Arg Asn Leu His Gln Gly Ser Asn Thr Leu Thr Thr
    530                 535                 540

Ala Ala Gly Thr Ile Ala Gly Thr Ala Gly Thr Val Glu Val Leu Gly
545                 550                 555                 560

Asp Gly Arg Trp Val His Leu Glu Gly Phe Gly Gly Tyr Ala Met Leu
                565                 570                 575

Asp Asp Ser Pro Leu His Val Leu Arg Glu Thr Arg Ser Gly Ser Trp
            580                 585                 590

Ser Gly Val Asn Ile Asn Gly Ser Ala Thr Val Gln Gln Arg Asn Phe
        595                 600                 605

Ala Thr Leu Tyr Val Asn His Gly Val Gly Pro Val Ala Gly Ser Tyr
    610                 615                 620

Ala Tyr Met Val Ala Pro Gly Ala Ser Val Asp Leu Thr Arg Lys Leu
625                 630                 635                 640

Leu Glu Gly Asn Lys Tyr Ser Val Ile Arg Asn Asp Ala Thr Ala Gln
                645                 650                 655

Ser Val Glu Phe Lys Thr Ala Lys Thr Thr Ala Ala Thr Phe Trp Lys
            660                 665                 670

Pro Gly Met Ala Gly Asp Leu Gly Ala Ser Gly Pro Ala Cys Val Val
        675                 680                 685

Phe Ser Arg His Gly Asn Glu Leu Ser Leu Ala Val Ser Glu Pro Thr
    690                 695                 700

Gln Lys Ala Ala Gly Leu Thr Leu Thr Leu Pro Glu Gly Thr Trp Ser
705                 710                 715                 720

Ser Val Leu Glu Gly Ala Gly Thr Leu Gly Thr Asp Ala Asp Gly Arg
                725                 730                 735

Ser Thr Leu Thr Leu Asp Thr Thr Gly Leu Ser Gly Lys Thr Lys Leu
            740                 745                 750

Ile Lys Leu Lys Arg
        755

<210>  2
<211>  342
<212>  PRT
<213>  Artificial

<220>
<223>  A partial polypeptide of chondroitin AC lyase

<220>
<221>  misc_feature
<222>  (4)..(10)
<223>  Xaa = Any amino acid

<220>
<221>  misc_feature
<222>  (12)..(45)
<223>  Xaa = Any amino acid

<220>
<221> misc_feature
<222> (47)..(50)
<223> Xaa = Any amino acid

<220>
<221> misc_feature
<222> (52)..(59)
<223> Xaa = Any amino acid

<220>
<221> misc_feature
<222> (61)..(109)
<223> Xaa = Any amino acid

<220>
<221> misc_feature
<222> (111)..(118)
<223> Xaa = Any amino acid

<220>
<221> misc_feature
<222> (120)..(172)
<223> Xaa = Any amino acid

<220>
<221> misc_feature
<222> (174)..(176)
<223> Xaa = Any amino acid

<220>
<221> misc_feature
<222> (178)..(179)
<223> Xaa = Any amino acid

<220>
<221> misc_feature
<222> (181)..(286)
<223> Xaa = Any amino acid

<220>
<221> misc_feature
<222> (288)..(341)
<223> Xaa = Any amino acid

<400> 1

Asn Trp Trp Xaa Xaa Xaa Xaa Xaa Xaa Xaa Arg Xaa Xaa Xaa Xaa Xaa
1               5                   10                  15

Xaa Xaa Xaa Xaa Xaa Xaa Xaa Xaa Xaa Xaa Xaa Xaa Xaa Xaa Xaa Xaa
            20                  25                  30

Xaa Xaa Xaa Xaa Xaa Xaa Xaa Xaa Xaa Xaa Xaa Xaa Xaa Gln Xaa Xaa
        35                  40                  45

Xaa Xaa Arg Xaa Xaa Xaa Xaa Xaa Xaa Xaa Xaa Asn Xaa Xaa Xaa Xaa
    50                  55                  60

Xaa Xaa Xaa Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Xaa Xaa Xaa
                85                  90                  95

Xaa Xaa Xaa Xaa Xaa Xaa Xaa Xaa Xaa Xaa Xaa Xaa Xaa His Xaa Xaa
            100                 105                 110

Xaa Xaa Xaa Xaa Xaa Xaa Tyr Xaa Xaa Xaa Xaa Xaa Xaa Xaa Xaa Xaa
        115                 120                 125

Xaa Xaa Xaa Xaa Xaa Xaa Xaa Xaa Xaa Xaa Xaa Xaa Xaa Xaa Xaa Xaa
    130                 135                 140

Xaa Xaa Xaa Xaa Xaa Xaa Xaa Xaa Xaa Xaa Xaa Xaa Xaa Xaa Xaa Xaa
145                 150                 155                 160

Xaa Xaa Xaa Xaa Xaa Xaa Xaa Xaa Xaa Xaa Xaa Xaa Arg Xaa Xaa Xaa
                165                 170                 175

Arg Xaa Xaa Asn Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Xaa Xaa
        195                 200                 205

Xaa Xaa Xaa Xaa Xaa Xaa Xaa Xaa Xaa Xaa Xaa Xaa Xaa Xaa Xaa Xaa
    210                 215                 220

Xaa Xaa Xaa Xaa Xaa Xaa Xaa Xaa Xaa Xaa Xaa Xaa Xaa Xaa Xaa Xaa
225                 230                 235                 240

Xaa Xaa Xaa Xaa Xaa Xaa Xaa Xaa Xaa Xaa Xaa Xaa Xaa Xaa Xaa Xaa
                245                 250                 255

Xaa Xaa Xaa Xaa Xaa Xaa Xaa Xaa Xaa Xaa Xaa Xaa Xaa Xaa Xaa Xaa
            260                 265                 270

Xaa Xaa Xaa Xaa Xaa Xaa Xaa Xaa Xaa Xaa Xaa Xaa Xaa Xaa Asn Xaa
        275                 280                 285

Xaa Xaa Xaa Xaa Xaa Xaa Xaa Xaa Xaa Xaa Xaa Xaa Xaa Xaa Xaa Xaa
    290                 295                 300

Xaa Xaa Xaa Xaa Xaa Xaa Xaa Xaa Xaa Xaa Xaa Xaa Xaa Xaa Xaa Xaa
305                 310                 315                 320

Xaa Xaa Xaa Xaa Xaa Xaa Xaa Xaa Xaa Xaa Xaa Xaa Xaa Xaa Xaa Xaa
                325                 330                 335

Xaa Xaa Xaa Xaa Xaa Trp
            340

## Description

### Field of the Invention

The present invention relates to chondroitin AC lyase (chondroitin AC lyase II, chondroitinase AC II) crystals having particular crystallographic characteristics. The present invention also relates to a protein having a chondroitin AC lyase activity and a particular amino acid sequence.

### Background Art

Glycosaminoglycans (GAGs) are the carbohydrate components of proteoglycans. They are highly negatively charged polysaccharides, made up of disaccharide repeating units of a substituted glucosamine or galactosamine attached through (1,4) linkages to a uronic acid. These disaccharide units are linked (1,3) or (1,4) into a polysaccharide chain (Ref. 1). The glucosamine/galactosamine are extensively sulfated. Their synthesis requires a concerted action of a large number of enzymes (Refs. 2 and 3). GAGs are a major component of the extracellular matrix (Ref. 4).

Glycosaminoglycans are degraded enzymatically by two type of enzymes: hydrolases or lyases (Ref. 5). The enzymatic mechanisms of hydrolases are well understood and occur either according to the retaining or inverting mechanism (Ref. 6). On the other hand, the molecular details of the enzymatic mechanism of GAG lyases are still poorly understood. A chemically plausible mechanism for the β elimination reaction has been proposed (Ref. 7), however, the constitution of the active site and the role of individual amino acids is not yet clear. A number of bacterial species synthesize GAG lyases, enzymes used to degrade and utilize glycosaminoglycans as a source of carbon in the bacterium's natural environment (Refs. 5 and 8). Polysaccharide lyases with known three-dimensional structures fall into two architectures: the right-handed parallel β-helix (pectate/pectin lyases, chondroitinase B, rhamnoglucuronan lyase etc.) and (α/α)₅ toroid *(Flavobacterium heparinum* chondroitin AC and chondroitin ABC lyases, bacterial hyaluronate lyases etc.) or (α/α)₆ toroid (alginate lyases). A catalytic mechanism has been proposed for pectate lyases, Ca²⁺-dependent enzymes (Ref. 9), but it remains to be seen if it applies to other lyases having the β-helix topology. Several plausible mechanisms have been proposed for the lyases with the (α/α)₅ toroidal fold, yet there is no clear evidence favoring one of them (Refs. 10 and 11). Recently Jedrzejas and co-workers (Ref. 12) indicated that their crystal structures of hyaluronidase-substrate complex provides a direct evidence for the role of histidine as the general base. However, the reported B-factors for many of the substrate atoms were 100 Å² (presumably refinement limit) and no electron density map for the substrate was shown. Therefore, their interpretation is questionable, leaving the question of the mechanism still unresolved. The nature of the group presumed to be necessary to neutralize the charge of the glucuronic acid carboxylic group is also not clear as these enzymes do not require Ca²⁺ ions and there are no positively charged group(s) in the vicinity of the uronic acid, as expected from the accepted chemical mechanism (Ref. 7).

Glycosaminoglycan degrading enzymes with defined specificity have found widespread applications as analytical tools for the analysis of structure of glycosaminoglycans and other polysaccharides (Ref. 13). Chondroitin AC lyases are frequently used for this purpose. These enzymes cleave the glycosidic bond on the non-reducing end of an uronic acid and use as a substrate either chondroitin-4-sulphate or chondroitin-6-sulphate but not dermatan sulfate. They also display a varied degree of activity toward hyaluronan. Enzymes from two sources: chondroitin AC lyase from *Arthrobacter aurescens* (ArthroAC) and from *Flavobacterium heparinium* (FlavoAC) are commercially available from Seikagaku Corporation and frequently used. The latter enzyme, FlavoAC, has been cloned and overexpressed in *F. heparinium* (Ref. 14) and in *E. coli* (Ref. 15). The inventors of the present invention have previously determined the three-dimensional structure of this enzyme on its own (Ref. 16) and in complex with several dermatan sulfate oligosaccharides (Ref. 10), and proposed alternative catalytic mechanisms. One of these alternative mechanisms was also put forward for hyaluronate lyases on the basis of complexes with products and molecular modeling (Refs. 11 and 17).

Although the purification and characterization of chondroitin AC lyase from *Arthrobacter aurescens* (ArthroAC) had been done many years ago (Refs. 18 to 20), and the enzyme have been used extensively as an analytical tool in glycosaminoglycan analysis, this enzyme has not xdbeen cloned and its amino acid sequence has not been determined. Only its amino acid composition and carbohydrate content were reported (Ref. 19).

In recent years, physiological activities of various kinds of GAGs are studied, especially molecular weight-specific physiological activities thereof also attract attention, and use thereof as drugs is also expected. Therefore, if the chondroitin AC lyase, one of the GAG degrading enzymes, can be provided in a highly purified crystallized state, it would be extremely useful in the production of chondroitin, chondroitin sulfate and so forth degraded into lower molecules as various analytical reagents, drugs and so forth or as drugs themselves. Moreover, if a protein having the chondroitin AC lyase activity can be provided, it would also have the same usefulness as mentioned above and is extremely advantageous in the production.

As described above, although it has already been reported so far that ArthroAC has been purified, and one reference reported that it has been even crystallized, crystals having unique characteristics such as the crystals of the present invention have remained unknown.

### Disclosure of the Invention

An object of the present invention is to provide chondroitin AC lyase (chondroitinase AC II) crystals having particular crystallographic characteristics and being useful as a tool for biochemical analyses or a means for producing GAGs degraded into lower molecular weight compounds. Another object of the present invention is to provide a protein having the chondroitin AC lyase activity, which has the aforementioned usefulness.

In order to achieve the aforementioned objects, the inventors of the present invention conducted various researches. As a result, they successfully obtained unique crystals of chondroitin AC lyase (ArthroAC) derived from an *Arthrobacter* bacterium *(Arthrobacter aurescens),* and identified the crystallographic characteristics of the crystals. Furthermore, they elucidated the primary structure of chondroitin AC lyase by structural analysis based on X-ray diffractometry, and compared it with the already elucidated primary structure of the chondroitin AC lyase (FlavoAC) derived from *Flavobacterium heparinum* to reveal that the former was an exo-type chondroitinase and the latter was an endo-type chondroitinase. They further identified amino acid residues in the enzyme playing a crucial role in the aforementioned degrading mechanism of ArthroAC.

Therefore, the present invention provides crystals of chondroitin AC lyase originated from an *Arthrobacter* bacterium, which belong to the monoclinic system space group P2₁ substantially with unit cell dimensions of a = 57.6 Å, b = 85.5 Å, c = 80.5 Å, β = 106.9° and contain one molecule in the asymmetric unit.

In the chondroitin AC lyase crystals of the present invention, the *Arthrobacter* bacterium is preferably *Arthrobacter aurescens.*

The present invention also provides a protein having an activity of chondroitin AC lyase and an amino acid sequence Asn-Trp-Trp- (Xaa)₇-Arg- (Xaa)₃₄-Gln- (Xaa)₄-Arg-(Xaa)₈-Asn- (Xaa)₄₉-His- (Xaa)₈-Tyr- (Xaa)₅₃-Arg- (Xaa)₃-Arg-(Xaa)₂- (Asn or Asp) - (Xaa)₁₀₆-Asn- (Xaa)₅₄-Trp (Xaa represents an arbitrary amino acid residue, and the numerals represent numbers of residues). The amino acid sequence of this protein is shown as SEQ ID NO: 2 in Sequence Listing.

As such a protein as mentioned above, preferred is a protein comprising an amino acid sequence corresponding to the amino acid numbers 124 to 465 of SEQ ID NO: 1 mentioned in Sequence Listing.

### Brief Description of the Drawings

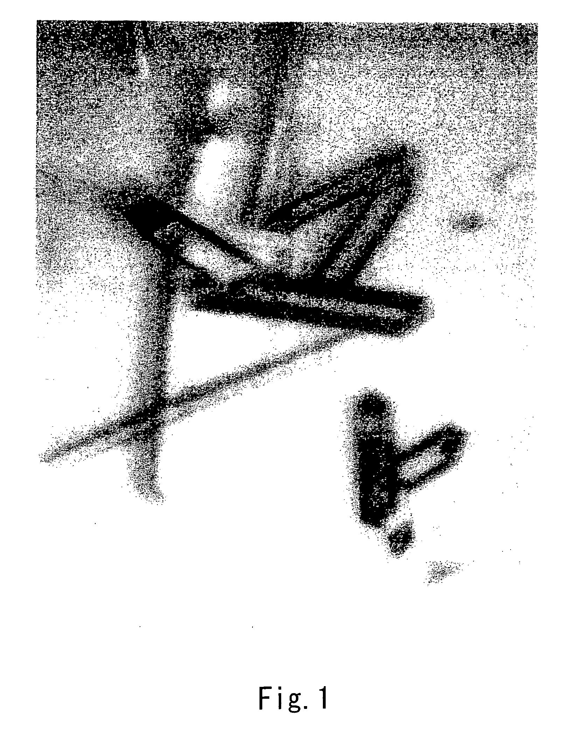

Fig. 1 is a microphotograph of chondroitin AC lyase II crystals.

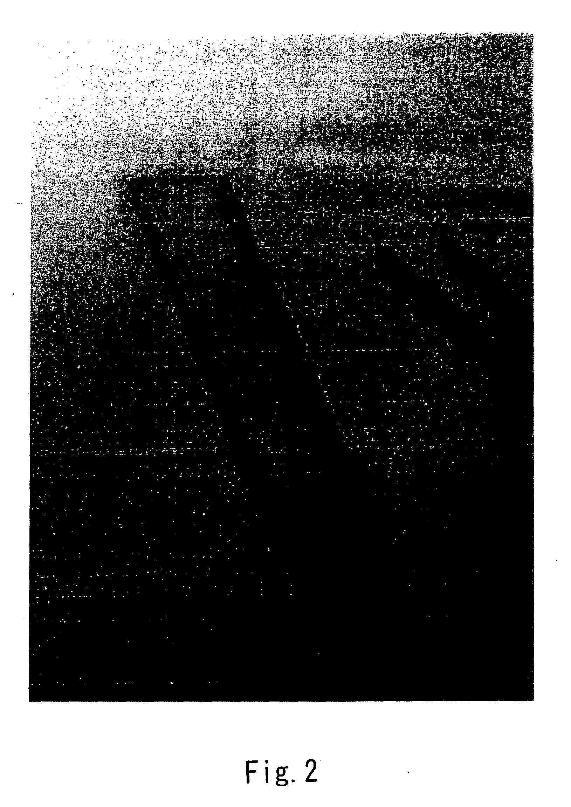

Fig. 2 is a microphotograph of chondroitin AC lyase II crystals.

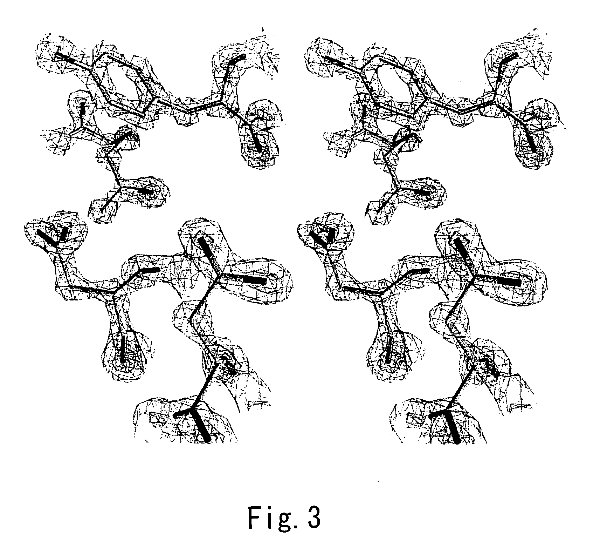

Fig. 3 shows a representative segment of the 3Fo-2Fc electron density map contoured at 3σ (green) and 6σ (magenta) levels. The map allows for differentiation of C, N and O atoms.

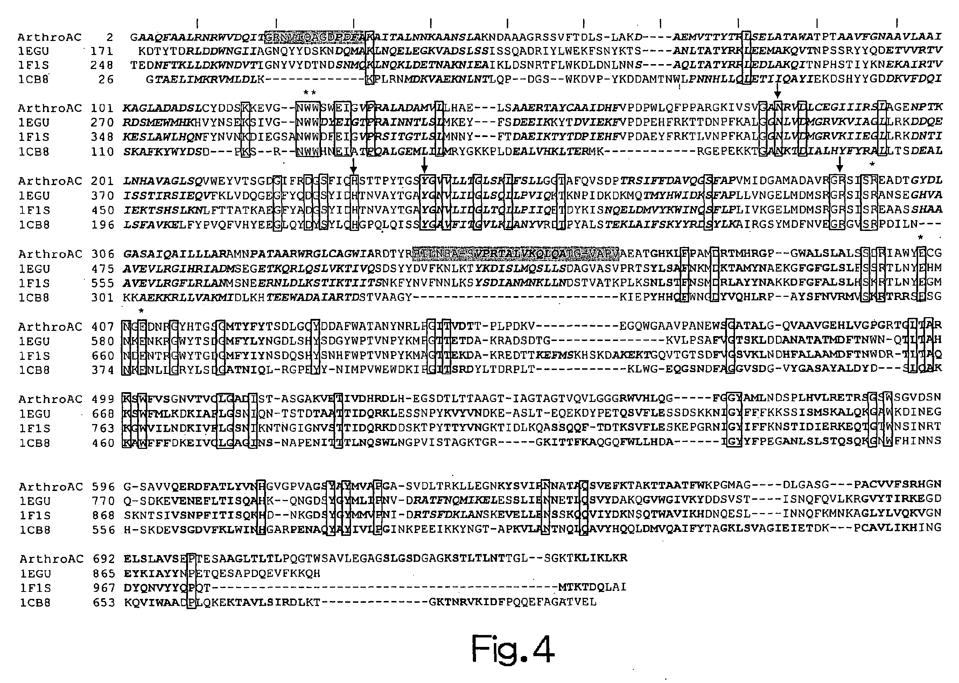

Fig. 4 shows structure-based sequence alignment for ArthroAC, FlavoAC and several hyaluronate lyases. Insertions in the ArthroAC sequence that close off the substrate binding site are marked in gray. Bold italic letters mark residues forming α-helices. Bold letters mark residues forming-β-strands. Residues conserved in all four proteins are enclosed in boxes. Arrows mark putative active site residues Asn180, His230, Tyr239, Arg293 and Glu404 (Glu404 is not indicated with arrow). This figure was prepared with programs MOLSCRIPT (Ref. 44) and Raster3d (Ref. 45). In the figure, G stands for a glycine residue, A for an alanine residue, V for a valine residue, L for a leucine residue, I for an isoleucine residue, S for a serine residue, T for a threonine residue, D for an aspartic acid residue, E for a glutamic acid residue, N for an asparagine residue, Q for a glutamine residue, K for a lysine residue, R for an arginine residue, C for a cysteine residue, M for a methionine residue, F for a phenylalanine residue, Y for a tyrosine residue, W for a tryptophan residue, H for a histidine residue, and P for a proline residue, respectively.

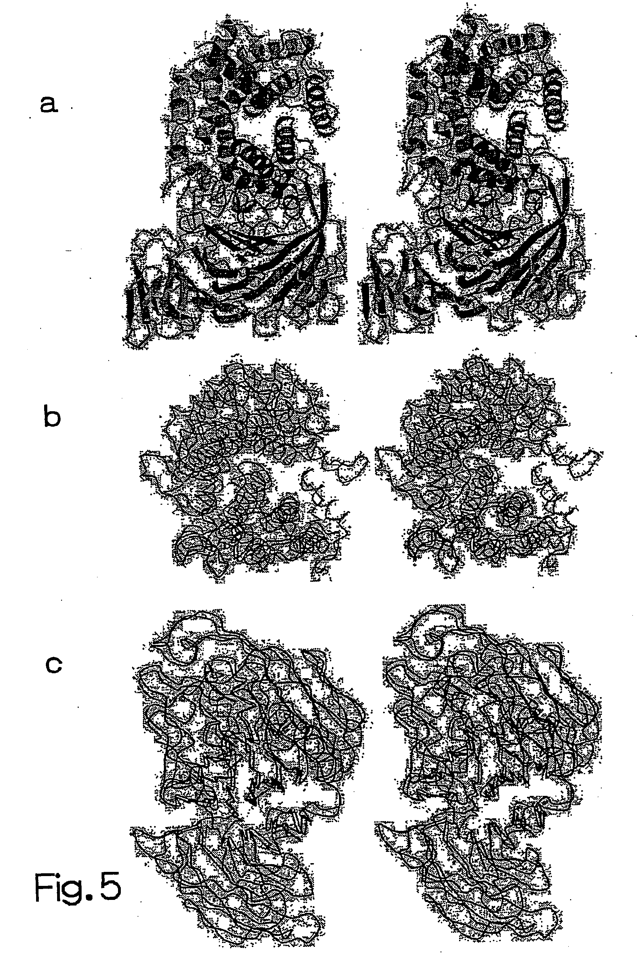

Fig. 5 includes the followings: a) Ribbon drawing of ArthroAC. The individual α-helical hairpins of the N-terminal (α/α)₅ toroid are painted in different colors. The individual β-sheets of the C-terminal domain are also painted in discrete colors. Insertions in ArthroAC blocking the cleft are painted in gray; b) Stereo view showing the superposition of the Cα traces of N-terminal domains of ArthroAC and FlavoAC. Insertions in ArthroAC blocking the cleft are painted in gray; c) Stereo view of the Cα traces of C-terminal domains of ArthroAC and FlavoAC.

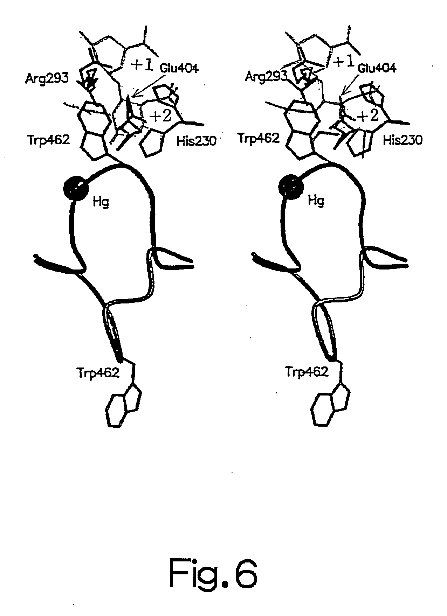

Fig. 6 shows a stereo view of the open and closed conformations of the 457-466 loop from the C-terminal domain. The location of the thimerosal Hg atom near the Cys405 in the open conformation is shown as ball. The sidechains of His230, Arg293, Glu404 and Trp462 are shown explicitly. The +2 and +1 sugars are also shown.

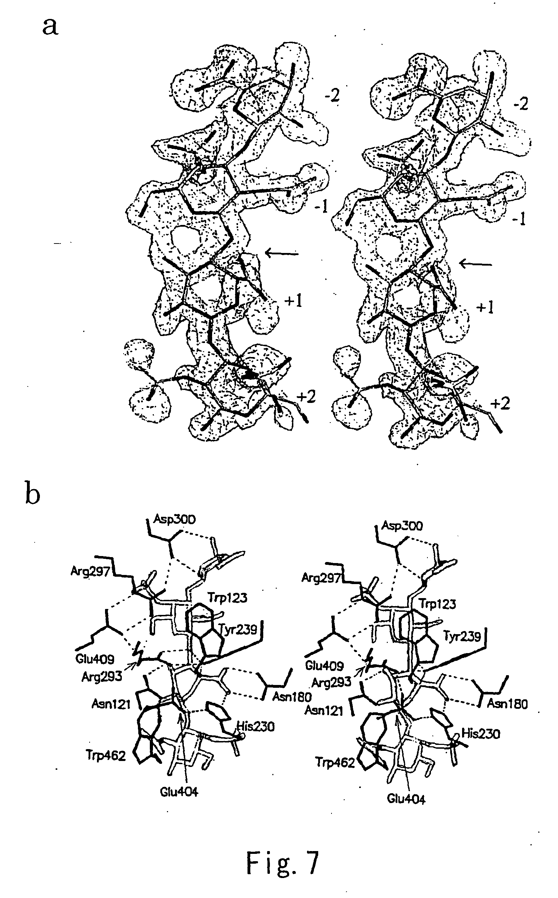

Fig. 7 shows a stereo view of the substrate binding site. a) Electron density in the omit map calculated without the substrate present and corresponding to the bound tetrasaccharide of chondroitin-4-sulfate is shown at 1.2σ level clearly indicating a distorted boat conformation of the +1 sugar unit; b) contacts of the tetrasaccharide to the neighboring sidechains. Hydrogen bonds are shown with dashed lines.

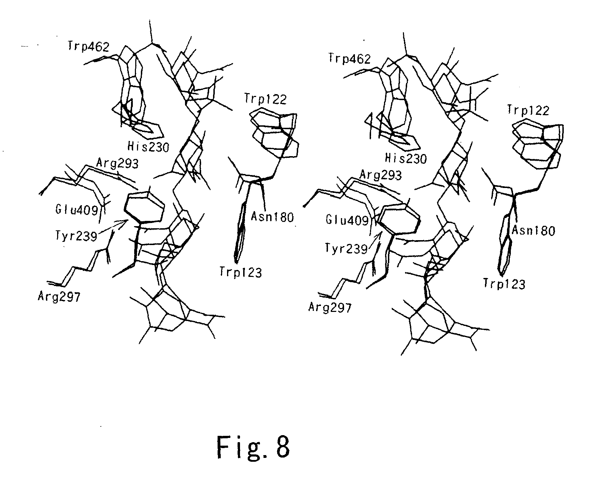

Fig. 8 shows a stereo view of the superposition of the complexes of *Arthrobacter* chondroitinase AC with chondroitin-4-sulfate tetrasaccharide (blue) and *Flavobacterium* chondroitinase AC Y234F mutant with chondroitin-4-sulfate tetrasaccharide (red).

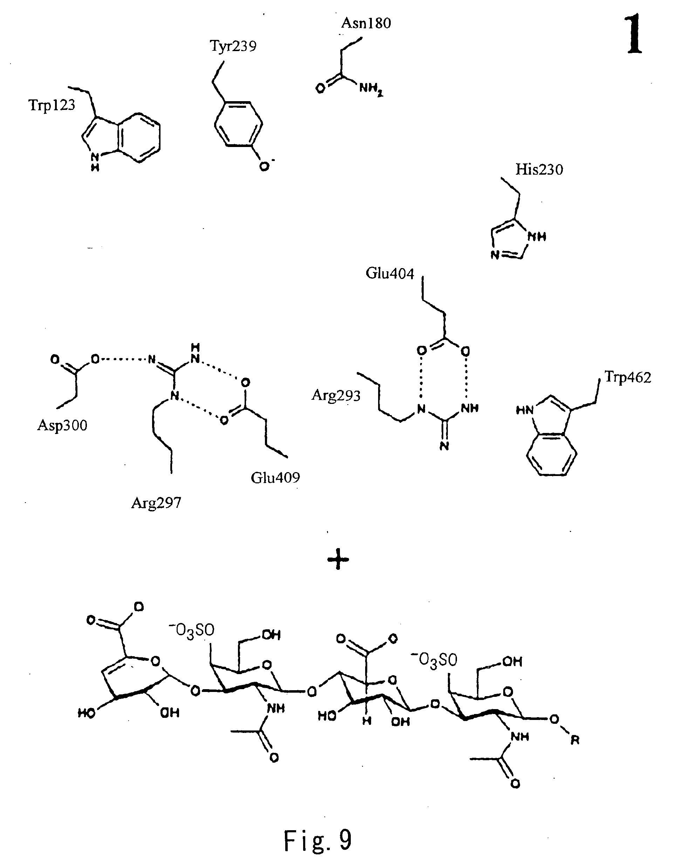

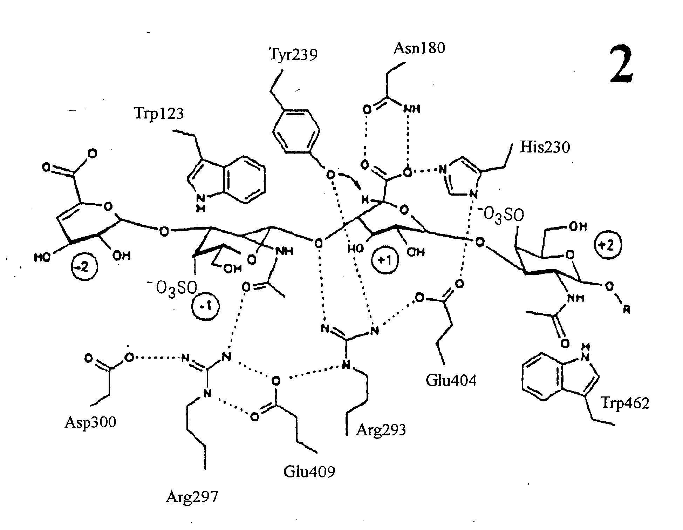

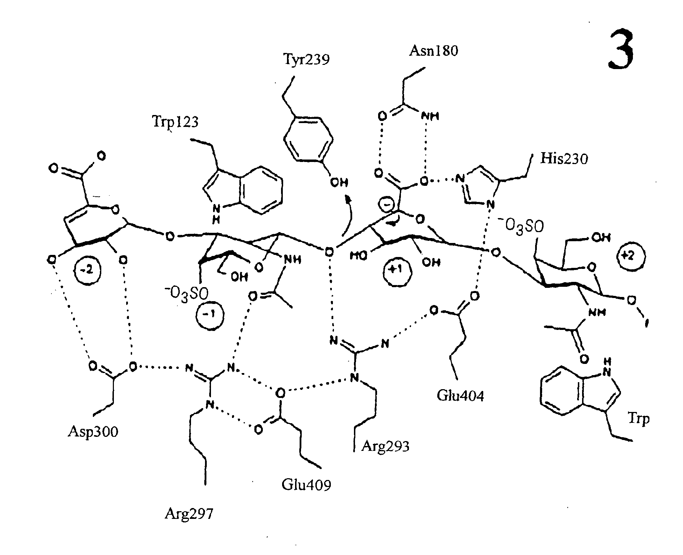

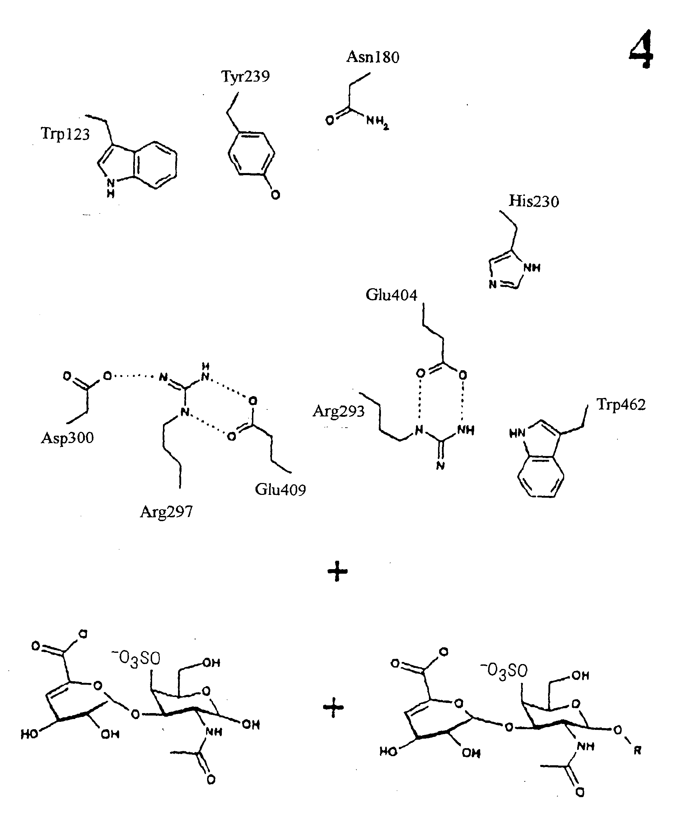

Fig. 9 shows a proposed catalytic mechanism. Closing and opening of the 457-466 loop, most likely associated with movements of the tips of two neighboring loops accompanies binding and release of the substrate. Binding of the substrate leads to the reorganization of the active site, and a deprotonation of Tyr239 influenced by the positively charged Arg293 and protonated His230 hydrogen-bonded to Tyr239. The OH⁻ of the phenyl ring acts initially as a general base abstracting the proton from C5 of glucuronic acid in +1 site. This proton is then donated to the 04 leaving group of the sugar in -1 site. The blocked end of the substrate binding canyon restricts the binding to the sugars at the non-reducing end of the glycosaminoglycan chain resulting in exolytic mode of action of ArthroAC.

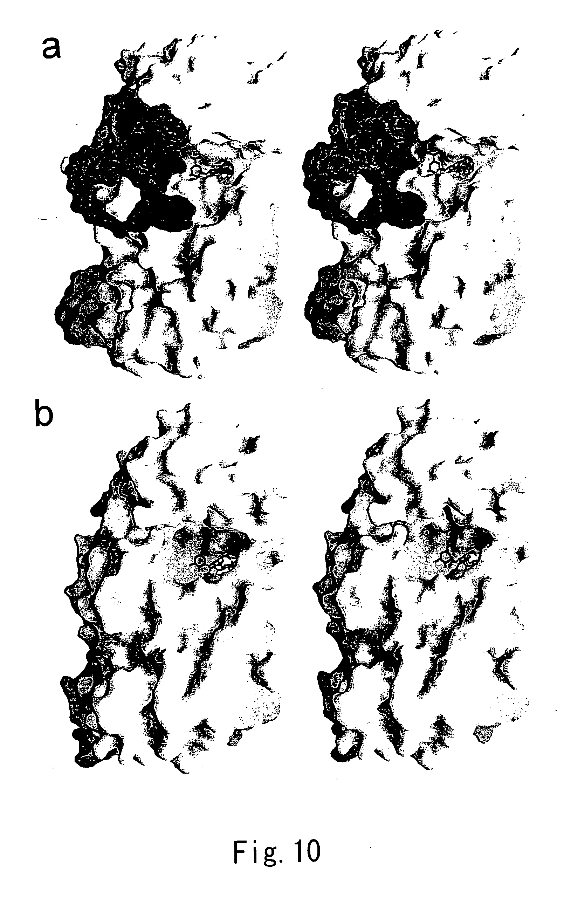

Fig. 10 shows stereo view of the molecular surface within the extended substrate binding site of a) ArthroAC. The insertion in the sequence capping the substrate binding site are shown in green; b) FlavoAC, with bound tetrasaccharides. Figure prepared with program GRASP (Ref. 46)

### Best Mode for Carrying out the Invention

Chondroitin lyases (EC 4.2.2.4 and EC 4.2.2.5) are glycosaminoglycan degrading enzymes that act as eliminases. Chondroitin lyase AC from *Arthrobacter aurescens* (ArthroAC) is known to act on chondroitin-4-sulfate and chondroitin-6-sulfate but not on dermatan sulfate. As other chondroitin AC lyases, it is also capable of cleaving hyaluronan.

The chondroitin AC lyase used for the crystals of the chondroitin AC lyase of the present invention itself is a known enzyme, and it has been already purified.

The chondroitin AC lyase used for the chondroitin AC lyase crystals of the present invention originates from an *Arthrobacter* bacterium. Although the *Arthrobacter* bacterium for obtaining the chondroitin AC lyase used for the chondroitin AC lyase crystals of the present invention is not particularly limited, it is preferably *Arthrobacter aurescens.*

The method for obtaining the chondroitin AC lyase from an *Arthrobacter* bacterium is not particularly limited either, and it can be obtained by using a method usually used for obtaining an enzyme form a bacterium. Specifically, after an *Arthrobacter* bacterium is cultured, the enzyme can be extracted from cells of the cultured bacterium and purified.

The *Arthrobacter* bacterium can be cultured by a usual method such as those described in J. Biol. Chem., 243(7), 1523-1535 (1968), Japanese Patent Laid-open Publication (Kokai) Nos. 62-122588, 2-57180 and so forth. Although the culture conditions are not particularly limited either, the bacterium is cultured, for example, in a medium containing meat or fish extract and polypeptone at a temperature of about 30 to 35°C for 12 hours to 5 days. In order to enhance expression of chondroitin AC lyase, it is preferable to add about 0.01% or more, preferably about 0.05 to 1%, of chondroitin sulfate or hydrolysate thereof to the culture medium.

The cells of the cultured bacterium are collected from the culture medium and suspended in a buffer having an approximately neutral pH, and the enzyme is extracted from the suspension. As the buffer having an approximately neutral pH, 1 to 100 mM phosphate buffer, Tris/hydrochloric acid buffer, acetate buffer and so forth having pH of 6.0 to 8.0 can usually be used. The cells are disrupted by ultrasonication or a treatment using a cell disruption apparatus (Dino Mill etc.) in such a buffer, and the enzyme is extracted as a bacterial cell extract containing chondroitin AC lyase, proteases, other enzymes, nucleic acids, proteins etc. Extraction efficiency of the extraction of chondroitin AC lyase from the cells may be increased by using a solution of a surfactant such as a buffer added with a surfactant.

After low molecular weight substances are removed by dialysis of the obtained cell extract against a buffer etc., the enzyme can be purified by using salting out, ammonium sulfate precipitation, various chromatography techniques etc.

Examples of the chromatography usable for the purification of chondroitin AC lyase include a chromatography treatment using a weak cation exchange resin and a strong cation exchange resin in combination. Examples of the weak cation exchange resin used here include cation exchange resins having carboxyalkyl groups such as carboxymethyl groups as cation exchange groups. Specific examples include polysaccharide derivatives having carboxymethyl groups as cation exchange groups (agarose derivatives, crosslinked dextran derivatives etc.), and examples of commercially available products include CM Sepharose, CM sephadex (both are trade names, Pharmacia) and so forth. Examples of the strong cation exchange resin include cation exchange resins having sulfoalkyl groups as cation exchange groups. Specific examples include polysaccharide derivatives having sulfoethyl groups, sulfopropyl groups etc. as the cation exchange groups (agarose derivatives, crosslinked dextran derivatives etc.), and examples of commercially available products include S Sepharose, SP Sepharose (trade names, Pharmacia), SP Sephadex (trade name, Pharmacia), SP TOYOPERL (trade name, TOSOH) and so forth. Examples of the chromatography treatment utilizing the aforementioned two kinds of cation exchange resins include the following method.

First, as the first chromatography treatment, a weak cation exchange resin is equilibrated with a buffer similar to that used for the extraction of the cells, for example, a buffer of pH 6.5 to 7.5 (e.g., 1 to 50 mM phosphate buffer, Tris/hydrochloric acid buffer, acetate buffer etc.), and the supernatant of the aforementioned cell extract is brought into contact with this resin so that the enzyme should be adsorbed on the resin. Then, if necessary, the cation exchange resin is washed with a saline solution (e.g., 20 to 25 mM NaCl solution and/or the aforementioned surfactant solution (e.g., 0.5% POELE solution)). An elution solution comprising the aforementioned buffer dissolving about 0.1 M sodium chloride is prepared and brought into contact with the resin to elute a fraction having the enzymatic activity. The elution method may be a concentration gradient method (gradient method) or a stepwise method. This chromatography treatment may be performed by a column method or a batch method. The obtained fraction is brought into contact with a strong cation exchange resin equilibrated with the same buffer so that the chondroitin AC lyase should be adsorbed on the resin, and if necessary, after this cation exchange resin is washed with a saline solution (e.g., 20 to 50 mM NaCl solution) and/or water, the chondroitin AC lyase is eluted for isolation with the same buffer as the buffer mentioned above containing 0 to about 0.5 M sodium chloride (e.g., phosphate buffer, Tris/hydrochloric acid buffer, acetate buffer etc.) by a concentration gradient method to obtain a purified enzyme solution. This chromatography treatment is preferably performed by a column method. The chromatography treatment described above using two kinds of cation exchange resins can also be performed in the reverse order.

Endotoxins, nucleic acids, proteases, others proteins and so forth as impurities are eliminated from the chondroitin AC lyase obtained as described above, and it shows a single band in electrophoresis (SDS-PAGE) and also shows a single peak in HPLC (gel filtration, cation exchange).

Furthermore, the chondroitin AC lyase is crystallized from the aforementioned purified enzyme solution. The method for crystallizing the purified chondroitin AC lyase is not particularly limited either, and a method usually used for crystallization of a protein can be used. For example, it is also possible to contact the chondroitin AC lyase with a polyether of a structure having hydroxyl groups at both ends (e.g., polyethylene glycol, polypropylene glycol etc.) by mixing to crystallize the chondroitin AC lyase. For example, polyethylene glycol (molecular weight: 4,000, 6,000, 8,000 etc.) is added to the chondroitin AC lyase solution, and the mixture is left standing at a temperature of from about room temperature to -4°C until crystals are formed. Further, the hanging drop vapor diffusion method, in which crystallization of the chondroitin AC lyase is attained by suspending a solution containing the chondroitin AC lyase and polyethylene glycol in a solution containing polyethylene glycol, is preferred. In particular, it is especially preferable to perform the crystallization by the method described in the examples mentioned later.

The crystals of the chondroitin AC lyase of the present invention obtained as described above are columnar crystals of the monoclinic system. The crystallographic characteristics thereof can be analyzed by X-ray diffractometry etc., and the inventors of the present invention obtained crystals of which three-dimensional structure could be determined with 1.25 Å resolution. The high resolution of this structure and a very good quality of the electron density maps of native enzyme and its complexes allowed them to deduce unambiguously the amino acid identity of 97% of the residues and to define the molecular details of the catalytic mechanism, which is common also to FlavoAC and hyaluronate lyases.

That is, as described in the examples mentioned later, the inventors of the present invention determined the three-dimensional crystal structures of ArthroAC in its native form as well as in complex with its substrates (chondroitin-4-sulfate tetrasaccharide, CS^{tetra} and hyaluronan tetrasaccharide) at resolution varying from 1.25 Å to 1.9 Å, and it was found that the crystals belonged to the monoclinic space group P2₁ substantially with cell dimensions a = 57.6 Å, b = 85.5 Å, c = 80.5 Å, β = 106.9° and contained one molecule in the asymmetric unit.

The primary sequence of ArthroAC has not been previously determined but it was possible to determine the amino acid sequence of this enzyme from the high resolution electron density maps.

The enzyme substrate complexes were obtained by soaking the substrate into the crystals for varying length of time and flash cooling the crystals. The electron density map for crystals soaked in the substrate for a short time (~2 min) showed clearly the substrate and indicated that the ring of the glucuronic acid in position +1 (refer to the definition mentioned below) is distorted into a boat conformation. This structure strongly supports the lytic mechanism where tyrosine (Tyr) 239 acts as a general base that abstracts the proton from the C5 position while asparagine (Asn) 180 and His230 neutralize the charge of glucuronate acidic group. Comparison of this structure with that of chondroitinase AC from *Flavobacterium heparinium* (FlavoAC) provides explanation for the exolytic mode of action for ArthroAC and the endolytic mode of action of FlavoAC.

### Examples

Hereafter, the present invention will be explained in more detail with reference to the following examples. However, the present invention is not limited to the following examples.

The descriptions in this specification follow the nomenclature introduced by Davies et al. (Ref. 21) and designate the sugars on the reducing end of the break with sign "+" and numbers increasing from the cleavage site, and the sugars on the non-reducing end with the "-" sign and numbers increasing away from the break. In this nomenclature the enzymes breaks the bond between sugars -1 and +1, the latter being an uronic acid.

### Culture of Arthrobacter aurescens and protein purification

To stimulate expression of ArthroAC, *Arthrobacter aurescens* was grown under the culture conditions as follows: medium (32.5 1) containing 0.4% peptone (Kyokuto Pharmaceutical Industry Co. Ltd., Tokyo), 0.4% Ehrlich's fish extract (Kyokuto Pharmaceutical Industry Co. Ltd., Tokyo) and 0.75% chondroitin sulfate C (Seikagaku Co., Tokyo); initial pH of 6.2; aeration rate of 1 v.v.m; agitation speed at 220 rpm; cultivation time - 24 h.

The enzyme was purified according to the previously described procedure (Ref. 20) with some modifications. Briefly, after bacterial cells were pelleted by centrifugation at 15,000 × g for 15 minutes, solid ammonium sulfate was added to the supernatant fluid up to 75% (w/v) saturation. At 4°C, 75 gram of the protein precipitate (50,000 unit) was sequentially dialyzed against distilled water with 0.02 M of acetate buffer (pH 5.2) and loaded on a SP-Sepharose (Amersham Bioscience Corp, Piscataway NJ) column (ϕ 2.6 × 70 cm), which was pre-equilibrated with the same buffer. After washing the column with several column volumes of buffer, the enzyme was eluted with a linear gradient from 0.02 to 0.3M of acetate buffer. Enzyme activity and protein amounts were monitored as previously described (Ref. 20). This chromatography procedure was repeated three times and the enzyme containing fractions were collected and concentrated by ultrafiltration (Ultrafilter Type P0200, molecular weight cut-off 20,000, Advantec, Tokyo Japan) under N₂. Finally, the enzyme was loaded on a Sephacryl S-200 HR gel filtration column (Amersham Bioscience Corp, Piscataway NJ) equilibrated with 0.01 M of acetate buffer (pH 5.6) and eluted with the same buffer. The fractions showing the highest specific activity and high purity by SDS-PAGE were pooled together and used in crystallization experiments.

### Oligosaccharide preparation

Chondrotin-4-sulfate tetrasaccharide (CS^{tetra}), dermatan sulfate hexasaccharide (DS^{hexa}) and hyaluronan tetrasaccharide were prepared and characterized as previously described (Ref. 10). Briefly, chondroitin-4-sulfate from bovine trachea and dermatan sulfate from porcine intestinal mucosa were subjected to controlled depolymerization using chondroitin ABC lyase and the reactions were terminated prior to completion by boiling for 5 min. Each oligosaccharide mixture was separated on a Bio-Gel P6 column and fractions consisting of tetrasaccharides and hexasaccharides were collected. These mixtures were further fractionated by strong-anion-exchange high performance liquid chromatography, single oligosaccharides were obtained, and their purity confirmed by capillary electrophoresis and their structures by MS and NMR analyses. These structures were: CS^{tetra}, ΔUAp (1→3)-β-D-GalpNAc4S (1→4)-β-D-GlcAp(1→3)-α, β-D-GalpNAc4S and DS^{hexa}, ΔUAp (1→ [3]-β-D-GalpNAc4S (1→4)-α-L-IdoAp (1→)₂3) -α, β-D-GalpNAc4S (where ΔUAp is the unsaturated sugar residue, 4-deoxy-α-L-threo-hex-4-enopyranosyluronic acid; IdoAp, idopyranosyluronic acid; GlcAp, glucopyranosyluronic acid; GalNp, 2-deoxy-2-aminogalactopyranose; S, sulfate; and Ac, acetate).

### Protein crystallization and data collection

Needle-shaped crystals of this protein have been reported many years ago (Ref. 20), however they were not characterized. The enzyme was crystallized by the hanging drop vapor diffusion method in drops containing 2 µl of protein (10 mg/ml) and 2 µl of reservoir solution (23% [w/v] PEG 8000, 0.1 M phosphate buffer pH 6.4, 0.4 M NH₄Ac, 10% [v/v] glycerol) suspended over 1 ml of reservoir solution. Small crystals appeared within a few days. In order to obtain larger crystals suitable for data collection, the macroseeding technique was used. A small crystal was washed and transferred to a fresh protein-containing drop with the precipitant concentration lowered to 21% [w/v]. Large, well ordered crystals grew within 3-5 weeks. Microphotographs of the chondroitin AC lyase II crystals obtained by the above procedure are shown in Figs. 1 and 2.

The crystals belong to the monoclinic space group P2₁ with cell dimensions a = 57.6 Å, b = 85.5 Å, c = 80.5 Å, β = 106.9° and contain one molecule in the asymmetric unit. Prior to data collection the crystals were immersed for 10 s in a cryoprotecting solution containing 22.5% [w/v] PEG 8000, 0.1 M phosphate buffer pH 6.4, 0.4 M NH₄Ac and 20% [v/v] glycerol, mounted in nylon loop and flash cooled in a cold stream of N₂ gas to 100°K. These crystals diffracted to 1.3Å resolution at the synchrotron. Data were collected at the X8C beamline at Brookhaven National Laboratory.

Crystals of the protein complexed with thimerosal were obtained by an overnight soaking of native crystals in the cryoprotectant solution containing an additional 2 mM of the Hg salt. Presence of this heavy-atom derivative caused an increase of the c-axis by 1.7 Å and resulted in cell dimensions: a = 57.4 Å, b = 85.3 Å, c = 82.2 Å, β = 105.8°. These crystals showed a significant degree of non-isomorphism with the native crystals. Datasets at three wavelengths were collected in the MAD regime.

### Enzyme-substrate complexes

To obtain complex of ArthroAC with its substrates, the inventors of the present invention resorted to a series of soaks of the native crystals in cryo-protecting solution containing the substrate for times ranging from 30 s to 10 h (Table 2). Native crystals were soaked in the cryo-protecting solution containing 5 mM of CS^{tetra} or HA^{tetra} for a specified length of time then flash frozen in cold nitrogen gas stream (100°K) on the detector's goniometer and used immediately for data collection. There was no significant change in cell dimensions as compared to the crystals of native ArthroAC.

Diffraction data for all the crystals were collected at the X8C beamline, NSLS, Brookhaven National Laboratory, using the Quantum-4 CCD detector. The highest resolution data, 1.25 Å, were obtained from the native crystal soaked for 10 h in 5 mM of CS^{tetra}. Data processing and scaling was performed with HKL2000 (Ref. 22). Collected data are shown in Tables 1 and 2.

### Structure determination

The structure of ArthroAC was solved from the Hg-containing thimerosal derivative of ArthroAC. Datasets were collected at three wavelengths in the Multiwavelength Anomalous Diffraction (MAD) experiment. Analysis of these datasets using the program SOLVE (Ref. 23) revealed three Hg sites in the asymmetric unit. These sites were used to calculate experimental phases to a resolution of 1.3 Å and resulted in the overall figure of merit (FOM) of 0.33 to 1.3 Å. Electron density modification performed with the program RESOLVE (Ref. 24) assuming solvent content of 0.4 led to a significant increase of the FOM (0.45 at 1.3 Å) and substantially improved the electron density map. Approximately 80% of the protein main chain was built automatically using program RESOLVE. Additional fragments of the mainchain and many sidechains were built manually using the program O (Ref. 25). Since the primary sequence of the protein was unknown, the amino acid type for each residue was selected to fit the experimental electron density map. At 1.3 Å resolution most of the assignments were unambiguous and nearly the entire chain was traced in the initial map. This initial sequence assignment was adjusted during the progress of refinement as the electron density features improved. Initial refinement was performed with the program CNS, version 1.1 (Ref. 26) and the model was manually rebuilt using the program O. Subsequently, refinement was continued using the program REFMAC5, version 5.1.08 (Ref. 27). During refinement of this model, 1% of the reflections were set aside for the calculation of Rfree. Water molecules were initially added automatically with the program CNS and subsequently updated and corrected by visual inspection of the difference map. The final model of the thimerosal-soaked crystal has been refined to an R-factor of 0.134 and Rfree of 0.155 at 1.3 Å resolution. The high quality of the electron density map allowed them to distinguish between the C, N and O atoms for most of the sidechains of Asn, Gln and His. This model contains 753 residues (Gly2 to Arg754) and corresponds to the amino acid sequence of ArthroAC mentioned in the alignment shown in Fig. 4. The amino acid numbers referred to in this specification are based on the numbers used for this sequence unless otherwise specified. The current model contains 754 residues (Pro4 to Arg757) and shown as SEQ ID NO: 1 in Sequence Listing with different numbering for amino acid numbers.

### Refinement of native protein and enzyme-substrate complexes

The model of Hg-bound ArthroAC was taken as a starting point for refinement of the crystal structure of the native protein. Refinement of all models was performed with the program REFMAC5, version 5.1.08. For monitoring of Rfree during refinement 1% of reflections were set aside. The electron density map showed that one loop had a substantially different conformation and was manually rebuilt with the program O. This model was refined at 1.35 Å resolution to a final R-factor of 0.130 and Rfree of 0.175. The model contains residues 2 to 754, 1029 water molecules and one Na⁺ ion. A strong feature near the catalytic residues was modeled as a phosphate ion.

This model in turn was used to determine the structures of all the complexes of chondroitin AC lyase with chondroitin 4-sulfate tetrasaccharide (CS^{tetra}) with different soaking time (30 s, 2 min, 10 min, 35 min, 2 h, 4 h, 10 h) and of HA^{tetra} complex (2 min soaking time). It was clear from the height of the difference electron density that the occupancies of sugars in sites -2, -1, +1 and +2 differed systematically from structure to structure. Since all datasets were collected to high resolution and the electron density features corresponded well to the expected sugar types, it was assumed that their temperature factors are similar between different crystals but occupancies differ. Therefore the occupancies of each sugar ring of the substrate were adjusted to result in similar B-factors across different crystals and having similar values as the surrounding protein atoms. In case of HA^{tetra} complex only two sugar rings were visible in the difference electron density map, in positions -2 and -1 (reaction product). Full data of refinements is summarized in Table 3.

PROCHECK (Ref. 28) showed that all models have good geometry with no outliers. Coordinates of the native chondroitinase AC (ArthroAC^{nat}), the mercury derivative (ArthroAC^{Hg}), 2 min soaked complex with hyaluronan (ArthroAC^{HA}), and 30 s, 10 min and 10 h soaked complexes with CS^{tetra} (ArthroAC^{CStetra}) are deposited in the Protein Data Bank, RCSB.

### Amino acid sequence of ArthroAC

ArthroAC was purified from its natural host and the gene that encodes this protein has not been cloned. Despite wide commercial use of this enzyme its amino acid sequence had not been determined. Fortunately, the quality of the electron density map calculated at 1.3 Å resolution, and the availability of several models refined against independently collected datasets was sufficient for unambiguous assignment of the amino acid sequence for almost entire length of the protein.

Approximately 80% of the protein mainchain atoms were built automatically with the program RESOLVE and this was extended to over 95% by manual fitting into the experimental electron density map. The strongest electron density features in the experimental map were assigned as the sulphur atoms and the residue type was set to either cysteine or methionine, based on the shape of the electron density. Identification of the sulphur atoms was confirmed by inspection of the anomalous electron density map calculated with the experimental phases. A total of 7 cysteines and 10 methionines were identified. All tryptophan, histidine, phenylalanine, tyrosine and proline sidechains were clearly recognizable in the experimental electron density map. Moreover, in the interior of the protein (especially in highly-ordered β-sheet domain) side chains of nearly all residues were very well defined (Fig. 3). Initially, all residues shaped as aspartic acid (Asp) or asparagine (Asn) were modeled as Asp, all residues shaped as glutamic acid (Glu) or glutamine (Gln) were modeled as Glu, all residues that could be interpreted as valines, threonines or isoleucines without clearly visible CD1, were modeled as valine (Val).

Improvement of phase information during the refinement allowed improvement of the sequence assignments. Assignment of sidechains involved thorough analysis of the peak heights in the 2Fo-Fc, 3Fo-2Fc and Fo-Fc electron density maps, comparison of individual atomic temperature factors and inspection of potential hydrogen bonds between neighboring atoms performed independently for each of the refined complexes. This analysis allowed them to distinguish between C, N and O atoms, which in turn permitted them to establish the primary sequence of ArthroAC with a very high level of confidence. In addition, partially occupied alternate conformations for some sidechains could be identified.

After completion of the refinement, 730 residues (96.8%) were assigned with high confidence a specific residue type. The electron density indicates a possibility of at least one residue before the first well resolved residue, therefore number 2 was assigned for the first resolved residue. For additional 12 residues (Asn/Asp and Gln/Glu) the assignment was less certain and it is based primarily on the potential formation of hydrogen bonds. Only 11 residues, located in flexible loops exposed to solvent and with high thermal factors, could not be identified unambiguously (Fig. 4).

The program BLAST (Ref. 29) was used to identify sequences in the NCBI database that are homologous to the derived amino acid sequence of ArthroAC. These homologous proteins are hyaluronate, xanthan or chondroitin AC lyases. ArthroAC shows the highest sequence identity to various hyaluronan and xanthan lyases (38%) and a lower identity to chondroitin AC lyase from *F. heparinum* (24%).

### Overall fold of protein

The ArthroAC molecule has an overall α + β architecture and consists of two domains. The N-terminal α-helical domain contains thirteen α-helices, ten of which form an incomplete double-layered (α/α)₅ toroid as classified within the SCOP database (Ref. 30). There is a long, deep groove on one side of the toroid that forms the location of the active site and substrate-binding site. Three α-helices at the N-terminus precede the (α/α)₅ toroid and constrict the cleft on one side. Residues conserved across the sequences of proteins homologous to ArthroAC cluster in the area of this cleft. The C-terminal domain is composed almost entirely of β-strands arranged into four β-sheets. There is only one short α-helix within this domain (Fig. 5). The second domain can be subdivided into two subdomains - the first encompasses two large β-sheets and one short α-helix while the second subdomain is composed of the third and fourth β-sheet.

The overall fold of ArthroAC is very similar to that of chondroitinase AC from *Flavobacterium heparinium* and to bacterial hyaluronate lyases (Fig. 5). A difference in the part of the chain protruding into the cleft in the N-terminal domain observed between ArthroAc and FlavoAC has important consequences for the mode of action of these two enzymes (see below). Comparison of all available related structures shows that the C-terminal β-sheet domains are more similar to each other then are the N-terminal α-helical domains, and that the relative disposition on the N- and C-terminal domains differs somewhat from protein to protein.

### Structural changes upon binding Hg atoms

When crystals of native ArthroAC were soaked in a solution containing thimerosal, a change in unit cell dimension along the c-axis of 1.7 Å (2%) was observed, accompanied by a small increase in diffraction quality of the crystals. Comparison of the native and Hg-bound structures showed that binding of thimerosal to cysteine (Cys) 405 causes significant structural changes in the conformation of the 457-466 loop within the C-terminal domain (Fig. 6). This loop in the native crystal provides one wall of the substrate binding site and shields Cys405 from the solvent. For the Hg atom to approach Cys405 this loop has to switch into an "open" conformation, which makes the substrate binding site accessible to the substrate. Situated within this loop is tryptophan (Trp) 462, which participates in substrate binding by stacking against one of the sugar rings (see below). The open conformation is stabilized by the interactions of Trp462 with a symmetry-related molecule. These new interactions cause the observed changes in cell parameters, and also slightly improve crystal diffraction quality. While the open conformation is very well defined in the crystal due to specific crystal contacts, it is expected that this conformation represents one of many possible conformations available to this loop in the "open" state in solution.

The opening of the 457-466 loop leads to a rearrangement of three residues within the N-terminal α-helical domain in the center of the substrate binding site, namely histidine (His) 230, arginine (Arg) 293 and glutamic acid (Glu) 404 (Fig. 6). In the closed conformation, Arg293 forms salt bridges with Glu404 and Glu409 (one H-bond to each), with His230 hydrogen bonded to Glu404. The sidechain of Trp462 packs nearby and is less than 4 Å away from the guanidinium group of this arginine. In the "open" conformation of the 457-466 loop the Trp462 sidechain moves out of the cleft and the sidechains of Arg293 and His230 extend into the volume previously occupied by Trp462. The sidechain of Glu404 moves together with Arg293 now forming a salt bridge with two H-bonds, while the His230 and Glu404 H-bond as well as the salt bridge between Arg293 and Glu409 are no longer present.

### Substrate binding site

The structures of native and Hg-bound ArthroAC showed that opening and closing of the 457-466 loop occurs readily in the crystal. These crystals provide therefore a unique opportunity to follow, by X-ray diffraction, the interactions of the enzyme with its substrate. To accomplish this, the native crystals of ArthroAC were soaked with chondroitin-4-sulfate tetrasaccharide substrate for various times, ranging from 30 s to 10 h (Tables 2 and 3). At the end of each soak, the crystal was immediately flash frozen and diffraction data collected (resolution varying between 1.25 Å and 1.6 Å, Table 3). Hyaluronan tetrasaccharide was also used as a substrate with 2 min soaking time, and diffraction data were collected to 1.9 Å resolution. The structures were refined independently. In each case, the ArthroAC molecule was refined first, then the difference electron density map was inspected and appropriately interpreted, and the modeled substrate/product was included in the refinement. Several sugar units were clearly visible in each refined model. Even in the 30 s soak, the electron density for the entire tetrasaccharide substrate is clearly visible (Fig. 7 and Table 4). Indeed, the tetrasaccharide substrate is best defined in this dataset and the sugar rings have occupancy of 0.7 for the -1 and - 2 subsites and 0.6 for the +1 and +2 subsites. For the 10 h soak the sugar units in subsites -1 and -2 are clearly visible in the difference electron density map and refine with occupancy of 1.0, while the sugars at the +1 and +2 subsites have occupancy of ~0.25. In the complex of ArthroAC with hyaluronan tetrasaccharide, only the sugar units in subsites -1 and -2 were visible in the electron density, most likely corresponding to a disaccharide reaction product. The location and orientation of the sugar rings of this disaccharide is the same as the corresponding sugars of chondroitin sulfate substrate.

The oligosaccharide is bound within the groove of the N-terminal domain near its narrow end and makes contacts with the residues Asn121, Trp122, Trp123, Arg131, Gln166, Arg171, Asn180, His230, Tyr239, Arg293, Arg297, Asp300, Asn407 and Trp462 (Fig. 7). These residues correspond to the amino acid residues specified in the sequence contained in the protein of the present invention, and in the sequence shown as SEQ ID NO: 1, they correspond to Asn124, Trp125, Trp126, Arg134, Gln169, Arg174, Asn183, His233, Tyr242, Arg296, Arg300, Asn303, Asn410 and Trp465, respectively. Tryptophanes play an essential role in substrate binding. Two of these residues, Trp123 and Trp462, provide a stacking interaction with sugar units occupying positions +1 and -2, while Trp122 is aligned edge-on and forms a hydrogen bond with the bridging oxygen between the +2 and +1 units. The 4-O-sulfo groups of the substrate form several interactions with the protein. The 4-O-sulfo group of the +2 sugar makes a H-bonds to Gln166 and, through a bridging water molecule, to Asp219 and Gln229. The -1 sugar 4-0-sulfo group is positioned just above the guanidinium group of Arg297 and, in addition, forms H-bonds to Glu409 and Asn595 through a bridging water molecule. Both 4-O-sulfo groups contribute to substrate binding but do not add significantly to the specificity of substrate recognition.

The chondroitin sulfate tetrasaccharide used in these studies was obtained by the action of GAG lyases and contained an unsaturated ring at the non-reducing end, with a C4-C5 double bond (Ref. 10). The electron density for the -2 sugar corresponds very well to this unsaturated ring, with the C5 having sp² hybridization. A detailed list of all hydrogen bonds between the tetrasaccharide and the protein sidechains is listed in Table 5.

Most interesting from the viewpoint of the mechanism of catalysis is the conformation and interactions with the enzyme of the +1 glucuronic acid. The electron density shows very clearly that this ring assumes a distorted boat conformation with all ring substituents being equatorial. The carboxylic group at C5 is placed exactly opposite the sidechain of Asn180, whose OD1 and ND2 are clearly distinguished by their peak height in the electron density map. The distance between the amide ND2 and the carboxyl's O6Å is 2.9 Å, and that between carbonyl OD1 and carboxyl's O6B is 2.5 Å. This short 06B-OD1 distance suggests the existence of a low-barrier hydrogen bond between them (Ref. 31), which in turn would indicate that the glucuronic acid carboxylic group is protonated and therefore in a neutral state. The 06A, in addition to accepting a hydrogen bond from ND2 of Asn180, also forms a second, 2.90 Å long hydrogen bond with the NE2 of protonated His230. The geometry of hydrogen bonds involving the carboxylic group is very close to ideal. The distance from the potential nucleophile His230 NE2 to the C5 of +1 glucuronic acid is rather long at 4.04 Å. The hydroxyl groups at C2 and C3 are also firmly held through several hydrogen bonds. Atoms 02 and 03 are H-bonded to OD1 and ND2 of Asn121, respectively, while 02 is also H-bonded to ND2 of Asn407 (Fig. 7b).

Two other residues make crucial contacts with the substrate. The Tyr239 hydroxyl is within H-bonded distance of the 04 bridging oxygen between +1 and -1 sugars, and 05 of the +1 sugar ring. This hydroxyl of Tyr239 is also only 3.0 Å from the C5 of the glucuronic acid. Arg293 sidechain forms also a hydrogen bond to the bridging 04 and is 2.9 Å from the Tyr239 hydroxyl.

The electron density corresponding to the glucuronic acid carboxylic group showed not two but three bulges, with the third one having somewhat lower density. This shape was common to the density observed in all datasets. Since the crystallization solution contained 0.1 M phosphate, a phosphate was modeled with partial occupancy in the same location (Table 5). The anomalous Fourier map calculated with the model phases that excluded the tetrasaccharide and the phosphate showed a small peak at the position of the phosphate, confirming the model.

A strong peak in the electron density map was found in the proximity of the tetrasaccharide, surrounded by six oxygen atoms in a tetragonal bipyramidal coordination, with distances to the equatorial oxygens of 2.2-2.4 Å and to the axial oxygens of 2.7 Å. This peak was assumed to be a sodium ion. The equatorial ligands are carbonyls of His230 and Trp462 and two water molecules, while the axial ligands are the OG1 of Thr232 and a water molecule.

### Comparison with other GAG lyases

The deduced sequence of ArthroAC was aligned with the sequences of FlavoAC and several hyaluronate lyases (Fig. 4). Interestingly, the amino acid identity is higher between ArthroAC and hyaluronate lyases (up to 38.4%) then with the FlavoAC (24.7%). The activity of ArthroAC against chondroitin sulfate and hyaluronan was measured and, indeed, it was observed that the enzyme shows a significant activity toward hyaluronan. Structures of ArthroAC and FlavoAC can be superimposed with a root-mean-squares (rms) deviation of 1.4 Å for 468 Cα atoms (out of 750 residues). Structural comparison with FlavoAC complexed with the dermatan sulfate hexasaccharide and with its inactive mutant Tyr234Phe complexed with chondroitin tetrasaccharide (Ref. 10) shows that the mode of oligosaccharide binding is nearly identical, with the largest difference restricted to the uronic acid at the +1 site (Fig. 8). The sidechains making crucial contacts with the oligosaccharide are conserved in their type and position. The complex of FlavoAC with dermatan sulfate hexasaccharide inhibitor shows that the +1 iduronic acid adopts a chair conformation, with the C5 acidic group in an equatorial position, while the hydroxyl substituents to the ring are axial. The position of the +1 sugar carboxylic group in the structure of FlavoAC with bound dermatan and in ArthroAC with bound chondroitin is nearly the same, due to a distorted boat conformation of the glucuronate sugar in the latter, with the acidic group in a pseudoaxial orientation. However, while the hydrogen bonds to Asn180 and His230 of ArthroAC have almost perfect geometry, the corresponding H-bonds in the FlavoAC complex are less than ideal. In the FlavoAC (Y234F)-chondroitin sulfate tetrasaccharide complex, the glucuronate sugars remain in a chair conformation with all substituents to the ring being equatorial, but rotates so that the acidic group occupies the space vacated by the missing hydroxyl of Tyr234 (Ref. 10). As previously remarked, the Y234F FlavoAC tetrasaccharide complex does not represent the true conformation of the substrate in the enzyme's binding site.

### Catalytic mechanism

The enzymatic reaction carried out by GAG lyases is thought to proceed via abstraction of the C5 proton by a general base followed by proton donation by a general acid or a water molecule to the bridging 04, and concomitant β-elimination of the leaving group (Ref. 7). Recent kinetic analysis of the FlavoAC using a well defined synthetic substrate agrees with the predicted stepwise, as opposed to concerted mechanism (Ref. 32). A proposal for the mechanism of polysaccharide lyases formulated by Gacesa (Ref. 7) included the neutralization of the acidic group by a positively charged group to shift the equilibrium toward the enolate tautomeric form. Unlike polysaccharide lyases that adopt the β-helix fold, the structures of FlavoAC and hyaluronate lyases showed an absence of such positively charged group in the vicinity of the acidic group of uronate. Instead, an asparagine side-chain was found to face and H-borid the acidic group, leaving the issue of neutralization of the acidic group an open question. The structures of the complexes presented here suggest that Asn180 by forming a low-barrier hydrogen bond with this acidic group increases substantially its pKa and promotes its protonation (Refs. 33 and 34). This asparagine is aided by His230, which also forms a H-bond to the carboxylate group of uronic acid (Fig. 7) and is protonated in the complex, as judged from the hydrogen bond network.

On the basis of structural view of GAG lyases-oligosaccharide complexes several proposal have been put forward as to the identity of the general base and general acid participating in the reaction. Jedrzejas and coworkers (Ref. 11 and 12) proposed that the proton is abstracted by a nearby histidine residue (His230 in the present structure) and that another proton is donated to 04 by a tyrosine (Tyr239 here). They support this proposed role of the histidine as the general base by the distance between the histidine NE1 and C5 in their structures. The inventors of the present invention have carefully re-analyzed their data. Their first suggestion to the role of histidine (Ref. 11) reinforced in the subsequent paper (Ref. 35), is based on the structures of complexes where only the "-" sites are occupied. The +1 sugar is modeled and the NE1-C5 distance of ~4 Å is not an experimental value. This value corresponds to a standard van der Waals distance and seems to be too long for the proposed proton-abstracting role of the histidine. In the recent paper (Ref. 12), they claim the presence of the substrate in the crystals of wild type hyaluronidase upon soaking for several days in 10-50 mM solution of a hexasaccharide. The temperature factors quoted for the most of the substrate atoms are 100 Å², the limit of the refinement program. Since the electron density for the substrate has not been shown in their paper, the inventors of the present invention have calculated the difference map based on their deposited structure factors and coordinates (excluding the substrate). The inventors of the present invention observed relatively weak scattered density and were unable to place the oligosaccharide with any confidence. The inventors of the present invention therefore concluded that this density corresponds to a mixture of products with very low occupancy and can not be modeled with confidence. However, even according to their model the distance between the histidine NE1 and C5 is over 6A. Therefore, the inventors of the present invention conclude that there is no structural evidence in their work to support the proposed role of this histidine. What is more, His399Ala mutant of *S. pneumoniae* hyaluronate lyase shows still a significant 6% of the wild type enzyme activity (Ref. 11).

As mentioned above, ArthroAC shows significant activity towards hyaluronan, and the structure of a complex between the enzyme and HA^{tetra} after even a brief 2 min soak shows the presence of only two sugar units in subsites -1 and -2, likely the reaction product. This contrast with substantial occupancy of subsites +1 and +2 for chondroitin sulfate tetrasaccharide after similar soaking time and suggests that ArthroAC is more active toward hyaluronan than toward chondroitin sulfate. This result correlates well with a higher sequence similarity of ArthroAC to hyaluronate lyases than to FlavoAC (Fig. 4). In view of these findings and the identity of the catalytically important residues in ArthroAC and hyaluronate lyases, the possibility that the hyaluronan substrate bound in the active site of the crystallized wild type *S*. *pneumoniae* hyaluronate lyase for several days remains intact, as stipulated by Jedrzejas (Ref. 12), is rather unlikely.

Huang et al. (Ref. 10) considered three possible mechanistic scenarios, ultimately favoring one in which a tyrosine initially functioned as a general base and subsequently as a general acid. In a structurally related but sequence distant alginate lyase, the central role was also assigned to a tyrosine (Mikami et al. 2001). The kinetic measurements of Rye and Withers (Ref. 32) also strongly favor tyrosine as the catalytic residue.

The structure of the complex of ArthroAC with the chondroitin-4-sulfate tetrasaccharide substrate provides very strong support for the key role played by the sidechain of Tyr239 (Fig. 9). The high resolution structure of the complex clearly shows that substrate binding is associated with a deformation of the glucuronate sugar ring at the +1 site from a chair to a distorted boat. Such a change to a higher energy conformation of the sugar ring is not unusual and has been observed in other carbohydrate processing enzymes (Refs. 36 to 41). In the observed distorted boat conformation, the acidic group is pseudoaxial and is brought face-to-face in plane with the sidechain of Asp180 and in plane with the protonated His230 (Fig. 7b). Furthermore, the disposition the glucuronate acidic group relative to Asp180 and the very short distance to the carbonyl oxygen of Asn180 of 2.5 Å suggests that the acidic group is protonated and forms a low-barrier hydrogen bond with Asn180. In this complex Tyr239 forms a H-bond to the bridging 04 and its OH group is only 3.0 Å from the glucuronate C5 atom. Arg293 provides a crucial contribution through hydrogen bonding to the hydroxyl group of Tyr239 and to the bridging 04. In contrast, the other potential general base, His230 is protonated and the distance between its NE and the C5 atom (3.9 Å) is rather long to function in acid-based catalysis.

The comparison of open and closed conformations of ArthroAC provides a rationale for reconstruction of the progress of events during the catalytic cycle. In the absence of the substrate the 457-466 loop is mobile, with Trp462 pointing away from the binding site. The sidechain of His230 is fully exposed to the solvent and makes H-bonds only to ordered water molecules. The Arg293 and Glu404 sidechains form a salt bridge largely solvent exposed. In this conformational state Tyr239 makes only one H-bond to a water molecule. Binding of the substrate leads to a rearrangement of the residues in the vicinity of the active site. Trp462 swings toward the substrate, stacking against the sugar ring at the +2 site. Concomitantly, Arg293 and Glu404 move toward Tyr239, with Arg293 engaging in a second salt bridge with Glu409 and forming a hydrogen bond to Tyr239 and the 04 atom bridging between the -1 and +1 sites.

To fit the binding site, the sugar ring at the +1 site must adopt a higher energy distorted boat conformation that is compensated by the formation of strong hydrogen bonds between the now protonated acidic group of glucuronate and Asn180 and protonated His230. The proximity of Tyr239 to positively charged Arg293 and His230 (H-bond) lowers substantially pKa of this tyrosine. The inventors of the present invention postulate that the Arg293 and His230 play a role of activating this tyrosine, priming it for its role as a general base. Subsequently, Tyr239 abstracts the proton from C5 of glucuronic acid, which is less than 3 Å away. The inventors of the present invention propose that this proton is subsequently shuttled to the bridging 04 (general acid role) with a concomitant breaking of C4-O4 bond and the formation of a C4-C5 double bond. The product at +1 and +2 sites is the first to leave, accompanied by the opening of the 457-466 loop. The binding and release of substrate is likely assisted by the movement of two other loops, with Asn121 and Asn407 at their tips, to expedite access to -2 and -1 sites.

The conservation in FlavoAC and hyaluronate lyases of all residues critical for the above described mechanism supports the view that this is a common mechanism for this class of enzymes. The inventors of the present invention have previously postulated the opening of the loops surrounding the substrate binding site in FlavoAC that they deemed necessary to explain the endolytic action of this enzyme (Ref. 10). The structure of ArthroAC described here provides direct evidence for such conformational flexibility.

### Rationale for the exo vs. endolytic mode of action

Enzymatic characterization of ArthroAC showed that it acts as an exo-lyase, releasing disaccharides from the glycosaminoglycan substrate, while FlavoAC acts as an endo-lyase (Refs. 42 and 43). Comparison of the structures of these two enzymes provides a rationale for the observed differences in the mode of action. ArthroAC has two insertions in the N-terminal domain relative to the FlavoAC: about 15 residues (Arg20-Ser35) and 25 residues (Thr340-Gly363). These two segments form α-helices-containing loops near the N- and C-termini of the domain, which come together closing off a large part of the cleft running along the side of the (α/α)₅ toroidal N-terminal domain. These loops form a wall that converts the open cleft into a deep cave and precludes binding of an extended oligosaccharide. The size of the cave restricts the binding of the carbohydrate on the non-reducing end and can accommodate approximately 4 sugars (-2 to -4) (Fig. 10a), which correlates with the exolytic activity of this enzyme. Binding of a longer carbohydrate would require substantial rearrangement of these two loops and apparently does not occur frequently. The open cleft of the FlavoAC does not impose such a constraint and allows binding to the middle of a long carbohydrate (Fig. 10b).

### Industrial Applicability

The chondroitin AC lyase crystals provided by the present invention are chondroitin AC lyase crystals having unique crystallographic characteristics unknown so far and extremely high purity. Therefore, they are extremely useful as a regent for various analyses, means for the manufacture of chondroitin as a drug and so forth.

Further, by the present invention, the primary structure of the chondroitin AC lyase derived from *Arthrobacter aurescens* was determined, and it was found that it was an exo-type chondroitinase. Therefore, the chondroitin AC lyase crystals of the present invention are expected to be useful for the manufacture of condroitin sulfate or condroitin having a particular molecular weight and so forth, against the background that the molecular weight-specific physiological activities of GAGs attract attentions in recent years.

Furthermore, the amino acid residues constituting the substrate binding site of chondroitin AC lyase were elucidated, and a protein containing them is provided. Thus, usefulness of the protein is expected in the manufacture of condroitin sulfate or condroitin having a particular molecular weight or as a drug itself.

**Table 1. MAD data collection statistics.**

| | Hg - Peak | Hg - Inflection | Hg - Remote | Native |
|---|---|---|---|---|
| Wavelength | 1.005133 | 1.009078 | 0.997068 | 0.950000 |
| Resolution range | 40-1.3 | 40-1.3 | 50-1.4 | 50-1.3 |
| (Last shell) | (1.35-1.30) | (1.35-1.30) | (1.45-1.40) | (1.35-1.30) |
| R_{sym} | 0.066 | 0.067 | 0.067 | 0.086 |
| (Last shell) | (0.274) | (0.325) | (0.221) | (0.540) |
| Completeness, % | 98.4 | 98.8 | 99.0 | 96.4 |
| (Last shell) | (85.0) | (89.0) | (90.7) | (93.4) |
| No. of reflections | 876240 | 884015 | 721975 | 1294307 |
| Unique reflecfions | 182880 | 183424 | 147941 | 178066 |

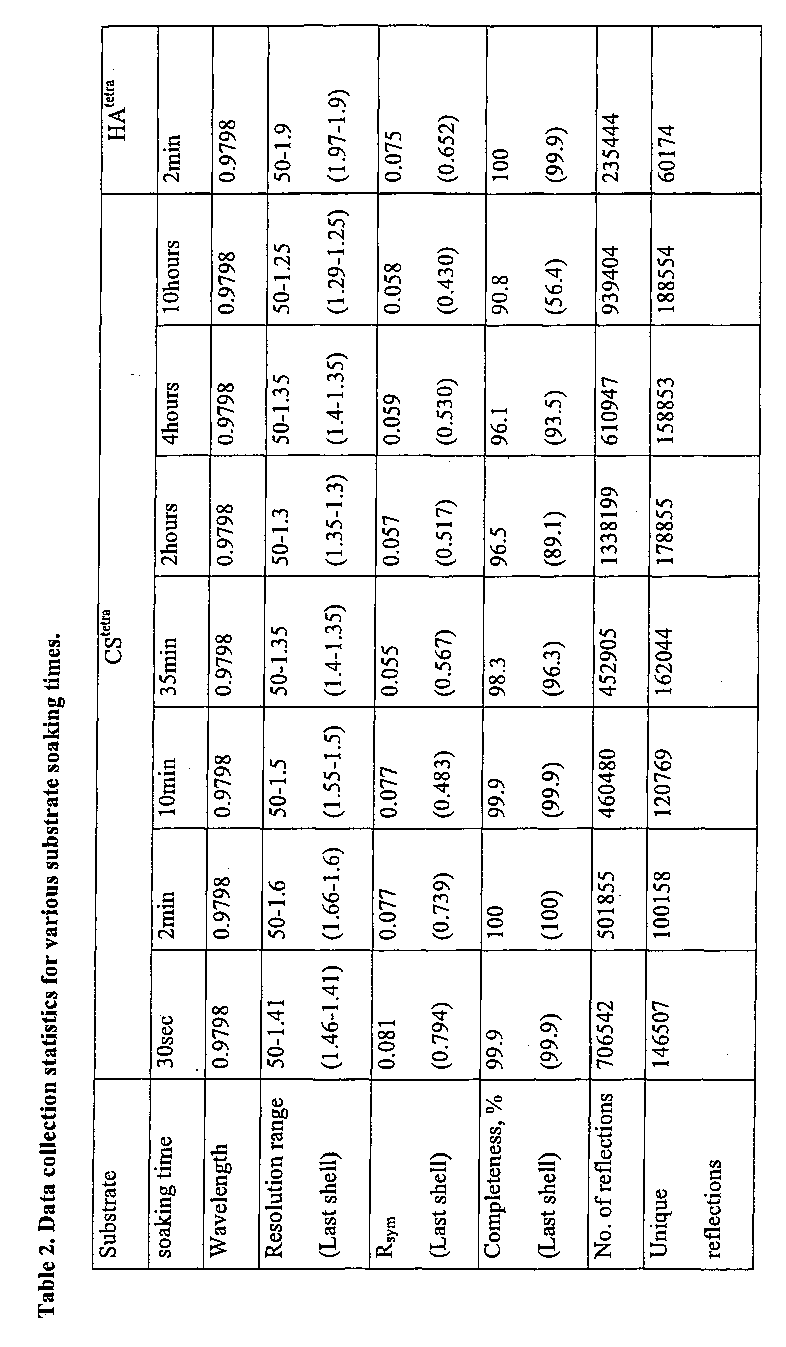

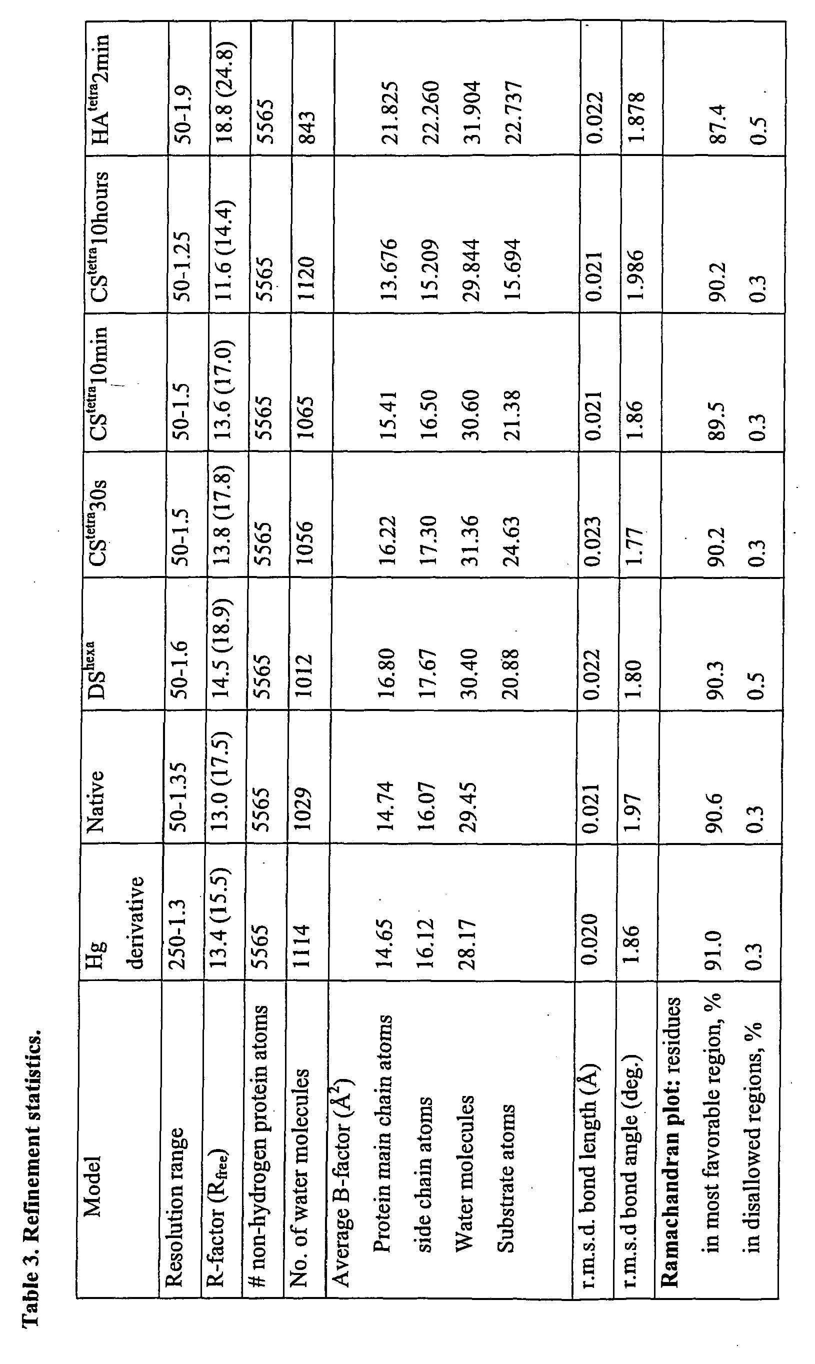

**Table 4. Occupancies of sugars in positions -2, -1, +1, +2 for different soaking times.**

| Soak time | | Site occupancy | | | | |
|---|---|---|---|---|---|---|
| | | -2 | -1 | +1 | +2 | Phosphate |
| native | | | | | | 1.0 |
| CS^{tetra} | 30s | 0.7 | 0.7 | 0.6 | 0.6 | 0.4 |
| | 2min | 0.7 | 0.7 | 0.5 | 0.5 | 0.5 |
| | 10min | 0.7 | 0.7 | 0.4 | 0.4 | 0.6 |
| | 35min | 0.7 | 0.7 | 0.4 | 0.4 | 0.6 |
| | 2h | 1.0 | 1.0 | 0.4 | 0.4 | 0.6 |
| | 4h | 1.0 | 1.0 | 0.3 | 0.3 | 0.7 |
| | 10h | 1.0 | 1.0 | 0.25 | 0.25 | 0.7 |
| HA ^{tetra} | 2min | 1.0 | 1.0 | - | - | 1.0 |

**Table 5. Hydrogen bonds between the substrate and the protein in CS^{tetra} complex.**

| Sugar number | Atom | Protein atom | Distance |
|---|---|---|---|
| 809 | O2 | OD2Asp300 | 2.86 |
| | O2 | NHlArg297 | 3.09 |
| | O3 | ODlAsp300 | 3.00 |
| | O6A | NHlArg131 | 3.03 |
| | O6B | NH2Arg131 | 2.85 |
| 810 | O7 | NH2Arg297 | 2.93 |
| | O7 | NH1Arg297 | 3.01 |
| | SO43 | NEArg297 | 3.17 |
| | SO44 | NH2Arg297 | 3.66 |
| 811 | O2 | ND1Asn407 | 3.16 |
| | O2 | ODlAsn121 | 2.66 |
| | O3 | ND2Asn121 | 2.83 |
| | O4 | NH2Arg293 | 2.88 |
| | O4 | OHTyr239 | 2.90 |
| | C5 | OHTyr239 | 2.89 |
| | O6A | NE2His230 | 2.75 |
| | O6A | ND2Asn180 | 2.88 |
| | O6B | OD1Asn180 | 2.51 |
| 812 | O1 | NH2Arg171 | 3.05 |
| | O3 | NE1Trp122 | 3.37 |
| | O7 | NH1Arg171 | 2.78 |
| | SO41 | NE2His230 | 2.75 |
| | SO43 | NE2Gln166 | 2.42 |

### References

1. lozzo, R. V. (1998) *Annu.Rev.Biochem.* 67, 609-652
2. Lidholt, K. (1997) *Biochem.Soc.Trans:* 25, 866-870
3. Prydz, K. and Dalen, K. T. (2000) *J. Cell Sci.* 113 Pt 2, 193-205
4. Scott, J. E. (1995) *J.Anat.* 187, 259-269
5. Ernst, S., Langer, R., Cooney, C. L., and Sasisekharan, R. (1995) *Crit. Rev. Biochem. Mol. Biol.* 30,387-444
6. McCarter, J. D. and Withers, S. G. (1994) *Curr.Opin.Struct.Biol.* 4, 885-892
7. Gacesa, P. (1987) *FEBS Lett.* 212, 199-202
8. Sutherland, I. W. (1995) *FEMS Microbiol.Rev.* 16, 323-347
9. Scavetta, R. D., Herron, S. R., Hotchkiss, A. T., Kita, N., Keen, N. T., Benen, J. A., Kester, H. C., Visser, J., and Jurnak, F. (1999) *Plant Cell* 11, 1081-1092
10. Huang, W., Boju, L., Tkalec, L., Su, H., Yang, H. O., Gunay, N. S., Linhardt, R. J., Kim, Y. S., Matte, A., and Cygler, M. (2001) *Biochemistry* 40, 2359-2372
11. Li, S., Kelly, S. J., Lamani, E., Ferraroni, M., and Jedrzejas, M. J. (2000) *EMBO J.* 19, 1228-1240
12. Jedrzejas, M. J., Mello, L. V., De Groot, B. L., and Li, S. (2002) *J.Biol.Chem.*
13. Linhardt, R. J., Galliher, P. M., and Cooney, C. L. (1986) *Appl.Biochem.Biotechnol.* 12, 135-176
14. Tkalec, A. L., Fink, D., Blain, F., Zhang-Sun, G., Laliberte, M., Bennett, D. C., Gu, K., Zimmermann, J. J., and Su, H. (2000) *Appl.Environ.Microbiol.* 66, 29-35
15. Pojasek, K., Shriver, Z., Kiley, P., Venkataraman, G., and Sasisekharan, R (2001) *Biochem.Biophys.Res.Commun* 286, 343-351
16. Féthière, J., Eggimann, B., and Cygler, M. (1999) *J.Mol.Biol.* 288, 635-647
17. Li, S. and Jedrzejas, M. J. (2001) *J.Biol.Chem.* 276, 41407-41416
18. Hiyama, K. and Okada, S. (1977) *J.Biochem. (Tokyo)* 82, 429-436
19. Hiyama, K. and Okada, S. (1975) *J.Biochem. (Tokyo)* 78, 1183-1190
20. Hiyama, K. and Okada, S. (1975) *J.Biol.Chem.* 250, 1824-1828
21. Davies, G. J., Wilson, K. S., and Henrissat, B. (1997) *Biochem.J.* 321 (Pt 2), 557-559
22. Otwinowski, Z. and Minor, W. (1997)*Methods Enzymol.* 276, 3 07-326
23. Terwilliger, T. C. and Berendzen, J. (1999) *Acta Crystallogr.* D55, 849-861
24. Terwilliger, T. C. (2000) *Acta Crystallogr.* D56, 965-972
25. Jones, T. A., Zhou, J. Y., Cowan, S. W., and Kjeldgaard, M. (1991)*Acta Crystallogr. A47,* 110-119
26. Brünger, A. T., Adams, P. D., Clore, G. M., DeLano, W. L., Gros, P., Grosse-Kunstleve, R W., Jiang, J. S., Kuszewski, J., Nilges, M., Pannu, N. S., Read, R. J., Rice, L. M., Simonson, T., and Warren, G. L. (1998) *Acta Crystallogr.* **D54**, 905-921
27. Murshudov, G. N., Vagin, A. A., and Dodson, E. J. (1997) *Acta Crystallogr.* **D53**, 240-255
28. Laskowski, R A., MacArthur, M. W., Moss, D. S., and Thornton, J. M. (1993) *J. Appl. Crystallogr.* 26, 283-291
29. Altschul, S. F., Madden, T. L., Schaffer, A. A., Zhang, J., Zhang, Z., Miller, W., and Lipman, D. J. (1997) *Nucleic Acids Res.* 25, 33 89-3402
30. Lo, C. L., Ailey, B., Hubbard, T. J., Brenner, S. E., Murzin, A. G., and Chothia, C. (2000) *Nucleic Acids Res.* 28, 257-259
31. Cleland, W. W., Frey, P. A., and Gerlt, J. A. (1998) *J.Biol.Chem.* 273,25529-25532
32. Rye, C. S. and Withers, S. G. (2002) *J.Am.Chem.Soc.* 124, 9756-9767
33. Cleland, W. W. (2000) *Arch.Biochem.Biophys.* 382, 1-5
34. Gerlt, J. A. and Gassman, P. G. (1993) *Biochemistry* 32,11943-11952
35. Ponnuraj, K. and Jedrzejas, M. J. (2000) *J. Mol.Biol.* 299, 885-895
36. Sidhu, G., Withers, S. G., Nguyen, N. T., McIntosh, L. P., Ziser, L., and Brayer, G. D. (1999) *Biochemistry* 38, 5346-5354
37. White, A. and Rose, D. R (1997) *Curr. Opin. Struct.Biol.* 7, 645-651
38. Sulzenbacher, G., Driguez, H., Henrissat, B., Schulein, M., and Davies, G. J. (1996) *Biochemistry* 35, 15280-15287
39. Kuroki, R, Weaver, L. H., and Matthews, B. W. (1993) *Science* 262, 2030-2033
40. Strynadka, N. C. and James, M. N. (1991) *J.Mol.Biol.* 220, 401-424
41. Strynadka, N. C. and James, M. N. (1996) *EXS* 75,185-222
42. Jandik, K. A., Gu, K., and Linhardt, R J. (1994) *Glycobiology* 4, 289-296
43. Capila, I., Wu, Y., Rethwisch, D. W., Matte, A., Cygler, M., and Linhardt, R J. (2002) *Biochim.Biophys.Acta* 1597, 260-270
44. Kraulis, P. J. *(1991)J.Appl.Crystallogr.* 24,946-950
45. Merritt, E. A. and Bacon, D. J. (1997) *Methods Enzymol.* 277, 505-524
46. Nicholls, A., Sharp, K. A., and Honig, B. (1991) *Proteins: Str.Func.Gen.* 11, 281-296

## Claims

1. Crystals of chondroitin AC lyase originated from an *Arthrobacter* bacterium, which belong to the monoclinic system space group P2₁ substantially with unit cell dimensions of a = 57.6 Å, b = 85.5 Å, c = 80.5 Å, β = 106.9° and contain one molecule in the asymmetric unit.

2. The crystals of chondroitin AC lyase according to claim 1, wherein the *Arthrobacter* bacterium is *Arthrobacter aurescens.*

3. A protein having an activity of chondroitin AC lyase and an amino acid sequence Asn-Trp-Trp-(Xaa)₇-Arg-(Xaa)₃₄-Gln- (Xaa)₄-Arg- (Xaa)₈-Asn- (Xaa)₄₉-His- (Xaa)₈-Tyr-(Xaa)₅₃-Arg- (Xaa)₃-Arg- (Xaa)₂- (Asn or Asp)- (Xaa)₁₀₆-Asn-(Xaa)₅₄-Trp (Xaa represents an arbitrary amino acid residue, and the numerals represent numbers of residues).
